(19) Europäisches Patentamt
European Patent Office
Office européen des brevets

(11) Veröffentlichungsnummer: **0 081 137 B1**

# (12) EUROPÄISCHE PATENTSCHRIFT

(45) Veröffentlichungstag der Patentschrift: **06.08.86**

(51) Int. Cl.⁴: **C 07 D 209/42**

(21) Anmeldenummer: **82110849.5**

(22) Anmeldetag: **24.11.82**

## (54) Verfahren zur Herstellung von Indolderivaten, deren Verwendung als wertvolle Zwischenprodukte und neue 4-Hydroxyindole.

(30) Priorität: **28.11.81 DE 3147276**

(43) Veröffentlichungstag der Anmeldung: **15.06.83 Patentblatt 83/24**

(45) Bekanntmachung des Hinweises auf die Patenterteilung: **06.08.86 Patentblatt 86/32**

(84) Benannte Vertragsstaaten: **AT BE CH DE FR GB IT LI LU NL SE**

(56) Entgegenhaltungen:
**DE - A - 2 145 573**

**CHEMICAL ABSTRACTS, Band 74, Nr. 21, 24. Mai 1971, Seite 388, Nr. 111846c, Columbus, Ohio, USA R. MARCHELLI, et al.: "Hydroxylation of indole-3-carboxylic acid"**
**CHEMICAL ABSTRACTS, Band 72, Nr. 3, 19. Januar 1970, Seite 313, Nr. 12470c, Columbus, Ohio, USA R. MARCHELLI et al.: "Preparation and properties of the hydroxyindole-3-carboxylic acids"**

(73) Patentinhaber: **Boehringer Mannheim GmbH, Sandhofer Strasse 116, D-6800 Mannheim 31 (DE)**

(72) Erfinder: **Michel, Helmut, Ziegelgasse 2a, D-6800 Mannheim 31 (DE)**
Erfinder: **Ofenloch, Roland, Hibiscusweg 2, D-6143 Lorsch (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur Herstellung von Indolderivaten der allgemeinen Formel I

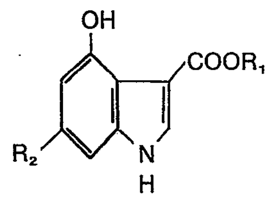

(I),

in der

$R_1$ Wasserstoff oder $C_1$–$C_6$ Alkyl und

$R_2$ Wasserstoff oder $C_1$–$C_6$ Alkyl

bedeuten.

Es wurde gefunden, dass das neue Verfahren in einfacheren Operationen und mit grösserer Reinheit zu wertvollen Zwischenprodukten führt, welche zur Herstellung von Verbindungen mit nützlichen pharmazeutischen Eigenschaften benötigt werden, z.B. Aminopropanole mit β-blockierenden Eigenschaften zur Behandlung von Angina pectoris und anderen Herzerkrankungen.

Verbindungen der Formel I, in welcher $R_2$ Wasserstoff bedeutet, sind literaturbekannt und wurden bereits nach einem anderen Verfahren hergestellt (Can. J. Chem. 47, 4375 [1969]).

In loc. cit. wird 4-Benzyloxyindol in die Magnesiumverbindung überführt, mit Chlorameisensäureethylester umgesetzt, einer verlustreichen chromatographischen Reinigung unterzogen und anschliessend hydrogenolytisch debenzyliert. Das als Ausgangsmaterial benötigte 4-Benzyloxyindol muss in vielstufiger Synthese aus einem 1,2,3-trisubstituierten Benzolderivat hergestellt werden.

Das erfindungsgemässe Verfahren zur Herstellung der Verbindungen der allgemeinen Formel I ist dadurch gekennzeichnet, dass man eine Verbindung der allgemeinen Formel II

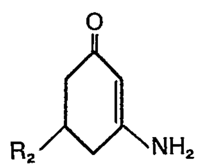

(II),

worin

$R_2$ Wasserstoff oder eine niedere Alkylgruppe darstellt, mit einer Verbindung der allgemeinen Formel III

$$R_3-CH_2-CO-COOR_1 \quad \text{(III)},$$

worin

$R_1$ Wasserstoff oder eine niedere Alkylgruppe und

$R_3$ einen reaktiven Rest darstellt,

umsetzt und

die erhaltenen Verbindungen der allgemeinen Formel V

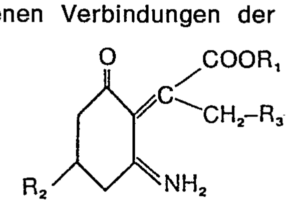

(V),

worin

$R_1$, $R_2$ und $R_3$ die oben genannte Bedeutung besitzen,

cyclisiert und anschliessend die erhaltenen Verbindungen der allgemeinen Formel IV

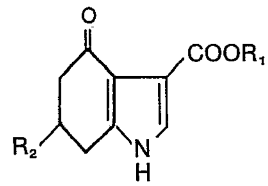

(IV),

worin

$R_1$ und $R_2$ die angegebene Bedeutung besitzen nach bekannten Methoden zu Verbindungen der Formel I dehydriert.

Unter Niederalkylgruppen der Substituenten $R_1$ und $R_2$ sind geradkettige oder verzweigte Gruppen mit 1–6 Kohlenstoffatomen zu verstehen. Bevorzugt sind jedoch die Methyl- und die Ethylgruppe. Als reaktive Reste $R_3$ kommen insbesondere Halogenatome, vorzugsweise das Chlor- oder Bromatom infrage.

Verbindungen der Formel IV sowie der Formel I′ sind neu und gleichzeitig Gegenstand der Erfindung.

Verbindungen der Formel I können nach bekannten Methoden, z.B. durch Verseifung und Decarboxylierung zu 4-Hydroxyindol, 4-Hydroxy-6-methylindol oder durch Aminolyse und Dehydratisierung zu 4-Hydroxy-3-cyanindol und 4-Hydroxy-3-cyan-6-methylindol umgewandelt werden, die durch Umsetzung mit Epichlorhydrin und Alkylaminoderivaten zu Aminopropanolen mit nützlichen pharmakologischen Eigenschaften führen (DE-OS 2 508 251, DE-OS 2 737 630, DE-OS 2 905 877 und EP-PS 0 045 911). Es können aber auch Verbindungen der Formel I direkt zu den Aminopropanolderivaten umgesetzt werden, worauf dann die erhaltenen Verbindungen nachträglich umgewandelt werden.

So wird z.B. aus 4-Hydroxy-3-cyanindol (EP-PS 0 045 910) das 4-(2,3-Epoxy-propoxy)-3-cyanindol erhalten, welches mit 2-(2-Allyloxyphenoxy)ethylamin zum pharmakologisch gut wirksamen 4-{2-Hydroxy-3-[2-(2-allyloxyphenoxy)ethylamino]propoxy}-3-cyanindol (Beisp. 3h von EP-PS 0 045 911) umgesetzt wird.

Verbindungen der Formel II und III sind literaturbekannt oder können nach allgemein bekannten Methoden erhalten werden.

Die Dehydrierung der Verbindungen der Formel IV führt man nach üblichen Methoden unter Schutzgasatmosphäre mit einem Edelmetallkatalysator durch, insbesondere Palladium.

Beispiel 1

3-Brom-2-(2-imino-6-oxo-cyclohexyliden)-propionsäure-ethylester

Zu 760 ml Brombrenztraubensäureethylester gibt man unter Rühren 111,1 g 1-Aminocyclohexen(1)-on-3 (Lit.: Arch. Pharm. 294, 763 (1961)) bei 30 °C. Danach wird 8 h auf 60 °C erwärmt. Nach Abkühlen wird mit 200 ml Essigester und 1000 ml Ether versetzt und abgesaugt. Nach Waschen mit

Ether verbleiben 247 g der Titelverbindung vom Schmp. 170–175 °C, d. s. 85% d. Th.

In analoger Weise erhält man wie in Beispiel 1 beschrieben

a) 3-Brom-2-(2-imino-4-methyl-6-oxo-cyclohexyliden)-propionsäureethylester
aus
1-Amino-5-methyl-cyclohexen(1)-on-3 und Brombrenztraubensäureethylester
vom Schmp. 165–170 °C, Ausbeute 92% d. Th.

Beispiel 2

3-Ethoxycarbonyl-4,5,6,7-tetrahydro-4-oxoindol

In 1 l n-Butanol werden 131 g Ammonacetat und 139,5 g wasserfreies Natriumacetat suspendiert, unter Rühren 247 g 3-Brom-2-(2-imino-6-oxo-cyclohexyliden)propionsäureethylester zugegeben und danach 6 h unter Rückfluss erhitzt. Nach Abkühlen giesst man in eine Lösung von 143 g $NaHCO_3$ in 1 l Wasser und rührt bei Raumtemp. über Nacht. Die organische Phase wird abgetrennt, mit Natriumsulfat getrocknet und im Vacuum eingeengt. Nach Anreiben mit Isopropanol, Absaugen und Trocknen verbleiben 86 g der Titelverbindung vom Schmp. 221–223 °C, d. s. 48% d. Th.

In analoger Weise wie in Beispiel 2 beschrieben erhält man:

a) 3-Ethoxycarbonyl-4,5,6,7-tetrahydro-4-oxo-6-methylindol
aus
3-Brom-2-(2-imino-4-methyl-6-oxo-cyclohexyliden)-propionsäureethylester, Ammonacetat und Natriumacetat
vom Schmp. 198–200 °C, Ausbeute 64% d. Th.

Beispiel 3

3-Ethoxycarbonyl-4-hydroxyindol

86,6 g 3-Ethoxycarbonyl-4,5,6,7-tetrahydro-4-oxo-indol
werden in 1 l Diethylenglycoldimethylether und 50 g 10proz. Palladiumkohle 20 h zum Rückfluss erhitzt. Nach Absaugen des Katalysators und Eindampfen im Vacuum wird mit Ether verrieben und abgesaugt. Es verbleiben 78 g der Titelverbindung vom Schmp. 151–153 °C, d. s. 90% d. Th.

In analoger Weise wie in Beispiel 3 beschrieben erhält man:

a) 3-Ethoxycarbonyl-4-hydroxy-6-methylindol
vom Schmp. 152–154 °C, d. s. 90% d. Th.

Beispiel 4

4-Hydroxyindol

44 g 3-Ethoxycarbonyl-4-hydroxyindol werden mit 19,2 g Natriumhydroxyd in 400 ml Ethanol und 100 ml Wasser 1,5 h am Rückfluss erhitzt. Die erhaltene Carbonsäure (Schmp. 234–236 °C, Zers.) wird in 250 ml Chinaldin mit 1 g Kupferpulver 30 Minuten zum Sieden erhitzt. Nach der üblichen Aufarbeitung erhält man 4-Hydroxyindol vom Schmp. 97–100 °C.

Beispiel 5

4-Hydroxy-3-cyanindol

5 g 3-Ethoxycarbonyl-4-hydroxyindol werden in 150 ml Methanol und 150 ml flüssigem Ammoniak 12 h auf 100 °C im Autoklaven erhitzt. Das erhaltene Amid wird in 50 ml Essigsäureanhydrid 5 h am Rückfluss erhitzt. Nach Verseifen der erhaltenen O-Acetylverbindung erhält man 4-Hydroxy-3-cyanindol vom Schmp. 206–207 °C.

**Patentansprüche**

1. Verfahren zur Herstellung von 4-Hydroxyindol-Derivaten der Formel I

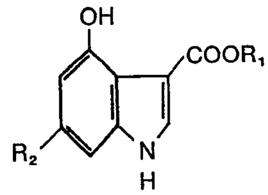

(I),

in der
$R_1$ Wasserstoff oder $C_1$–$C_6$ Alkyl und
$R_2$ Wasserstoff oder $C_1$–$C_6$ Alkyl
bedeuten, dadurch gekennzeichnet, dass man eine Verbindung der Formel II

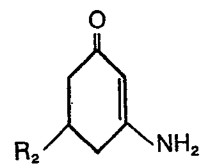

(II),

in der $R_2$ die oben angegebene Bedeutung hat, mit einer Verbindung der Formel III

$$R_3{-}CH_2{-}CO{-}COOR_1 \qquad (III),$$

in der $R_1$ die oben angegebene Bedeutung hat, $R_3$ einen reaktiven Rest darstellt, umsetzt, das erhaltene reaktive 2-(2-Imino-6-oxo-cyclohexyliden)-propionsäure-Derivat nach bekannten Methoden cyclisiert und die erhaltene Verbindung der Formel IV

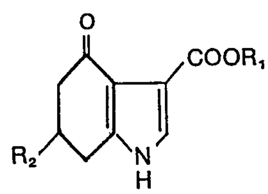

(IV),

in der
$R_1$ und $R_2$ die angegebenen Bedeutungen haben, dehydriert.

2. 4-Hydroxyindol-Derivate der Formel I′

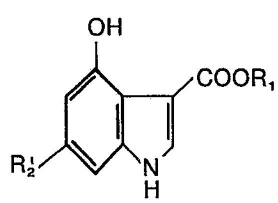

(I′),

in der
$R_1$ Wasserstoff oder $C_1$–$C_6$ Alkyl und
$R_2'$ $C_1$–$C_6$ Alkyl
bedeuten.

3. 4-Oxo-4,5,6,7-tetrahydro-indol-Derivate der Formel IV

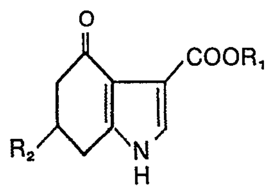

(IV),

in der

$R_1$ Wasserstoff oder $C_1$–$C_6$ Alkyl und

$R_2$ Wasserstoff oder $C_1$–$C_6$ Alkyl

bedeutet.

4. Verwendung der Verbindungen gemäss Anspruch 2 als Zwischenprodukte zur Herstellung von pharmakologisch wirksamen Aminopropanolen.

5. Verwendung der Verbindungen gemäss Anspruch 3 als Zwischenprodukte im Verfahren gemäss Anspruch 1 zur Herstellung von Verbindungen gemäss Formel I.

**Claims**

1. Process for the preparation of 4-hydroxyindole derivatives of the formula I

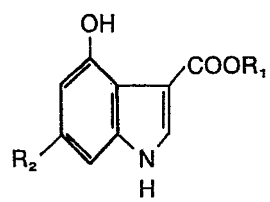

(I),

in which $R_1$ signifies hydrogen or $C_1$–$C_6$ alkyl and $R_2$ hydrogen or $C_1$–$C_6$ alkyl, characterised in that one reacts a compound of the formula II

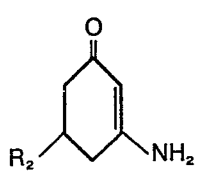

(II),

in which $R_2$ has the above-given meaning, with a compound of the formula III

$$R_3\text{–}CH_2\text{–}CO\text{–}COOR_1 \quad \text{(III)},$$

in which $R_1$ has the above-given meaning, $R_3$ represents a reactive residue, cyclises the 2-(2-imino-6-oxocyclohexylidene)-propionic acid derivative obtained according to known methods and dehydrogenates the compound obtained of the formula IV

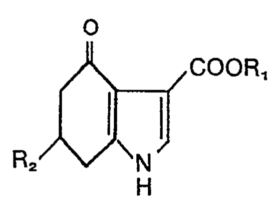

(IV),

in which $R_1$ and $R_2$ have the given meanings.

2. 4-Hydroxyindole derivatives of the formula I′

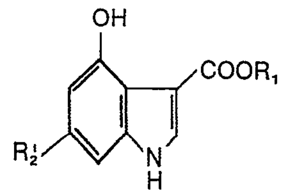

(I′),

in which $R_1$ signifies hydrogen or $C_1$–$C_6$ alkyl and $R_2'$ $C_1$–$C_6$ alkyl.

3. 4-Oxo-4,5,6,7-tetrahydroindole derivatives of the formula IV

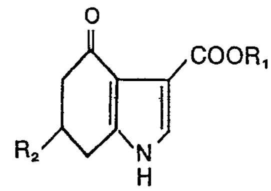

(IV),

in which $R_1$ signifies hydrogen or $C_1$–$C_6$ alkyl and $R_2$ hydrogen or $C_1$–$C_6$ alkyl.

4. Use of the compounds according to claim 2 as intermediate products for the preparation of pharmacologically-effective aminopropanols.

5. Use of compounds according to claim 3 as intermediate products in the process according to claim 1 for the preparation of compounds according to formula I.

**Revendications**

1. Procédé pour l'obtention de dérivés de 4-hydroxy-indole ayant pour formule I

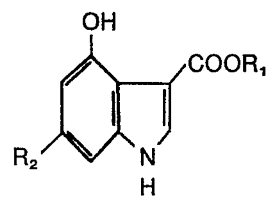

(I)

dans laquelle

$R_1$ est de l'hydrogène ou un alkyle $C_1$–$C_6$

$R_2$ est de l'hydrogène ou un alkyle $C_1$–$C_6$

caractérisé en ce que l'on soumet un composé de formule II

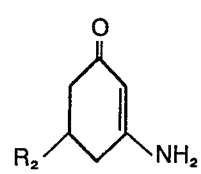

(II)

dans laquelle $R_2$ a la même signification que dans la formule précédente, à l'action d'un composé de formule III

$$R_3\text{–}CH_2\text{–}CO\text{–}COOR_1 \quad \text{(III)}$$

dans laquelle $R_1$ a la même signification que dans la formule précédente, $R_3$ désignant un reste réactif, en ce que l'on cyclise suivant des procédés connus le dérivé d'acide propionique 2-(2-imino 6-oxo-cyclohexylidène) obtenu et en ce que l'on soumet à une déshydrogénation le composé obtenu de formule IV

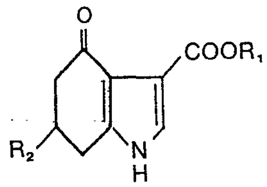

(IV)

dans laquelle

$R_1$ et $R_2$ ont la même signification que ci-dessus.

2. Dérivés du 4-hydroxy-indole de formule I'

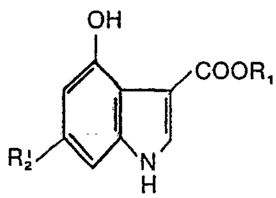

(I')

dans laquelle

$R_1$ désigne de l'hydrogène ou un alkyle $C_1$–$C_6$

$R_2'$ un alkyle $C_1$–$C_6$

3. Dérivés du 4-oxo 4,5,6,7-tétrahydro-indole de formule IV

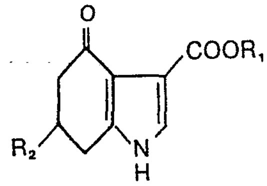

(IV)

dans laquelle

$R_1$ désigne de l'hydrogène ou un alkyle $C_1$–$C_6$

$R_2$ de l'hydrogène ou un alkyle $C_1$–$C_6$.

4. Utilisation des composés selon la revendication 2 comme produits intermédiaires pour la préparation d'aminopropanols doués de propriétés pharmacologiques.

5. Utilisation des composés selon la revendication 3 comme produits intermédiaires dans le procédé selon la revendication 1 pour la préparation de composés de formule I.